# EUROPÄISCHE PATENTSCHRIFT

(11) **EP 2 349 141 B1**
(45) Veröffentlichungstag und Bekanntmachung des Hinweises auf die Patenterteilung: **05.12.2012**
(21) Anmeldenummer: 09748041.2
(22) Anmeldetag: 23.10.2009
(51) Int. Cl.: A61F 6/04

(54) **KONDOM UND VERFAHREN ZU DESSEN HERSTELLUNG**
CONDOM AND METHOD FOR MANUFACTURE THEREOF
PRÉSERVATIF ET SON PROCÉDÉ DE FABRICATION

(30) Priorität: 24.10.2008 DE 102008053817
(43) Veröffentlichungstag der Anmeldung: 03.08.2011
(73) Patentinhaber: Zhang, Jian Hong, 13089 Berlin (DE)
(72) Erfinder: Zhang, Jian Hong, 13089 Berlin (DE)
(74) Vertreter: Hennings, Martin
(86) Internationale Anmeldenummer: PCT/EP2009/007740
(87) Internationale Veröffentlichungsnummer: WO 2010/046138

(56) Entgegenhaltungen:
- WO-A1-01/58394
- WO-A1-03/022188

## Beschreibung

Die Erfindung betrifft ein Kondom und Verfahren zu dessen Herstellung.

Bekannte Kondome bestehen aus einer an der Spitze geschlossenen und am entgegengesetzten Ende mit einer Wulst versehenen Schlauchhülle aus einem dünnwandigen flexiblen Isolationsmaterial, beispielsweise auf Basis von Naturharz - oder Kunststoff.

Als nächstliegender Stand der Technik sei die WO 01/583394 A1 genannt. Das darin beschriebene Kondom besteht aus einer an einem Ende mit einer Spitze und am entgegengesetzte Ende mit einer Wulst versehenen Schlauchhülle aus einem dünnwandigen flexiblen Isolationsmaterial, wobei die Schlauchhülle zwei leitfähige Abschnitte beinhaltet.

Aufgabe der vorliegenden Erfindung ist es, ein Kondom vorzuschlagen, das einerseits die Erektion fördert und andererseits das Lustgefühl während des Geschlechtsaktes erhöht. Weiterhin ist es Aufgabe der Erfindung, ein Verfahren zur Herstellung eines derartigen Kondoms vorzuschlagen.

Das erfindungsgemäße Kondom besteht aus einer an einem Ende mit einer Spitze und am entgegengesetzten Ende mit einer Wulst versehenen Schlauchhülle aus einem dünnwandigen flexiblen Isolationsmaterial mit hoher Isolationsfähigkeit. Die Schlauchhülle weist zwei leitfähige Abschnitte auf, die einen Kondensator bilden, der geeignet ist, elektrische Ladung zu speichern und zu einer Entladung während der Benutzung des Kondoms zu führen.

Vorzugsweise ist die Schlauchhülle mit Öffnungen versehen, wodurch sich der Kondensator während der Benutzung entladen kann. Die Öffnungen befinden sich an der Innenseite und/oder an der Außenseite der Schlauchhülle.

In einer Ausgestaltung des Kondoms weist der Kondensator zwei vor der Benutzung, insbesondere während der Herstellung zugängliche Abschnitte auf, die zur Aufladung des Kondensators dienen.

Vorzugsweise sind die leitfähigen Abschnitte aus einer leitfähigen Schicht oder leitfähigen Folie, insbesondere aus Gold hergestellt, insbesondere auf die Schlauchhülle aufgedampft.

Das Verfahren zur Herstellung eines erfindungsgemäßen Kondoms ist dadurch gekennzeichnet, dass die Schlauchhülle mit einer mit zwei leitfähigen Abschnitten versehen wird, die den Kondensator bilden.

Vorzugsweise werden die leitfähigen Abschnitte mittels Aufdampfen eines leitfähigen Stoffes hergestellt. Der leitfähige Stoff ist oder enthält beispielsweise ein Metall, insbesondere Gold.

Die Schlauchhülle wird an ihrer Innen- und/oder Außenseite mit Öffnungen versehen, die einen Kontakt der leitfähigen Abschnitte mit Körperflüssigkeiten ermöglichen.

Der Kondensator wird während der Herstellung aufgeladen, in einer Ausführungsform über die während der Herstellung zugänglichen Abschnitte.

Die Schlauchhülle besteht aus einem Material mit möglichst hoher Isolationsfähigkeit, um eine Entladung des Kondensators vor der Benutzung des Kondoms zu verhindern. Derartige Materialien werden heutzutage bereits bei modernen Kondensatoren in der Elektrotechnik/ Elektronik z.B. bei Supercaps verwendet.

Sofern das erfindungsgemäße Kondom die Funktionen eines herkömmlichen Kondoms, nämlich insbesondere die Schwangerschaftsverhütung, Verhinderung von Krankheitsübertragung, etc. ebenfalls erfüllen soll, ist die Spitze der Schlauchhülle geschlossen. Falls auf diese Funktionen verzichtet wird, muss die Spitze der Schlauchhülle nicht geschlossen sein.

Ein Ausführungsbeispiel der Erfindung wird im Folgenden unter Bezugnahme auf die beigefügte Zeichnung beschrieben.

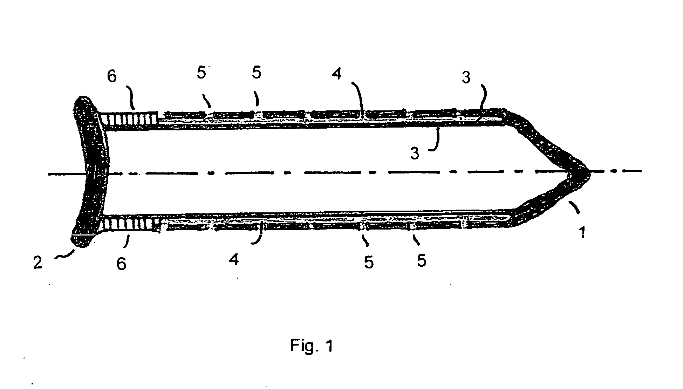

- Fig. 1: zeigt eine schematische Darstellung eines Kondoms in Seitenansicht.

Das Kondom besteht aus einer an der Spitze 1 geschlossenen und am entgegengesetzten Ende mit einer Wulst 2 versehenen Schlauchhülle aus einem dünnwandigen flexiblen Isolationsmaterial 3. Die Schlauchhülle weist zwei leitfähige Abschnitte 4 aus Gold auf, die einen Kondensator bilden, der geeignet ist, elektrische Ladung zu speichern und zu einer Entladung während der Benutzung des Kondoms zu führen.

Die Schlauchhülle ist mit Öffnungen 5 versehen, wodurch sich der Kondensator während der Benutzung entlädt und ein Reizstrom fließt. Die Öffnungen 5 befinden sich an der Außenseite der Schlauchhülle. Die leitfähigen Abschnitte 4 liegen dabei etwas weiter innen als die Oberfläche der Schlauchhülle. Dadurch wird eine vorzeitige Entladung während der Herstellung und vor der Benutzung des Kondoms verhindert. Selbst bei einem zusammengerollten Kondom berühren sich die leitfähigen Abschnitte 4 nicht, so dass auch in diesem Zustand keine Entladung erfolgt. Um die Gefahr des vorzeitigen Entladens zu verringern, können die leitfähigen Abschnitte 4 auch versetzt entlang einer gedachten längsseitigen Linie angeordnet sein. Um die Haltbarkeit der Ladung während einer langen Zeit zu erhöhen, besteht die Schlauchhülle aus einem nichtleitenden Material mit sehr hohem spezifischen elektrischen Widerstand. Die Entladung findet erst während der Benutzung des Kondoms statt, wenn die leitfähigen Abschnitte 4 über Körperflüssigkeiten elektrisch miteinander verbunden sind.

Die Öffnungen 5 besitzen in diesem Ausführungsbeispiel einen Durchmesser zwischen 1 µm und 10 µm. Sinnvoll können Durchmesser bis 1 mm sein.

In diesem Ausführungsbeispiel sind auf der oben und unteren Seite jeweils 7 Öffnungen 5 an der Außenseite der Schlauchhülle in etwa gleichmäßigem Abstand angeordnet. Vorzugsweise sind etwa 5 bis 10 Öffnungen 5 jeweils auf der Ober- und Unterseite angeordnet, insbesondere, wenn die Ober- und Unterseite jeweils den beiden leitfähigen Abschnitten 4 zugeordnet sind. Statt der gleichmäßigen Beabstandung der Öffnungen 5 ist es auch möglich, die Öffnungen 5 lediglich im hinteren Bereich, also nahe der Wulst 2 und/oder lediglich im vorderen Bereich, also nahe der Spitze 1 anzuordnen.

Die Öffnungen 5 befinden sich vorzugsweise an einer Stelle, die möglichst weit von der Stelle entfernt ist, an der sich die leitfähigen Abschnitte 4 am dichtesten gegenüberstehen.

In einer weiteren Abwandlung ist es auch möglich, die Öffnungen 5 an der Innenseite der Schlauchhülle oder an Innen- und Außenseite der Schlauchhülle zu platzieren. Um die Kapazität des Kondensators zu erhöhen, ist es ebenfalls möglich, die leitfähigen Abschnitte 4 auch innerhalb der Wulst 2 weiterzuführen, um so die Fläche der leitfähigen Abschnitte 4 zu erhöhen.

Der Kondensator weist des Weiteren zwei während der Herstellung zugängliche Abschnitte 6 auf, die zur Aufladung des Kondensators dienen.

Bei der Herstellung des erfindungsgemäßen Kondoms wird die Schlauchhülle mit zwei leitfähigen Abschnitten 4 versehen, die den Kondensator bilden. Die leitfähigen Abschnitte 4 werden mittels Aufdampfen von Gold auf den Isolationsmaterial 3 der Schlauchhülle hergestellt. Die Schlauchhülle wird an ihrer Außenseite mit Öffnungen 5 versehen, so dass die leitfähigen Abschnitte 4 von außen zugänglich sind.

Der Kondensator wird während der Herstellung aufgeladen, in diesem Ausführungsbeispiel über die während der Herstellung zugänglichen Abschnitte 6.

## Patentansprüche

1. Kondom bestehend aus einer an einem Ende mit einer Spitze (1) und am entgegengesetzten Ende mit einer Wulst (2) versehenen Schlauchhülle aus einem dünnwandigen flexiblen Isolationsmaterial (3), **dadurch gekennzeichnet, dass** die Schlauchhülle zwei leitfähige Abschnitte (4) beinhaltet, die einen Kondensator bilden, der geeignet ist, elektrische Ladung zu speichern und zu einer Entladung während der Benutzung des Kondoms zu führen.

2. Kondom nach Anspruch 1, **dadurch gekennzeichnet, dass** die Schlauchhülle mit Öffnungen (5) versehen ist, wodurch sich der Kondensator während der Benutzung entladen kann.

3. Kondom nach Anspruch 2, **dadurch gekennzeichnet, dass** sich die Öffnungen (5) an der Innenseite und/oder an der Außenseite der Schlauchhülle befinden.

4. Kondom nach einem der Ansprüche 1 bis 3, **dadurch gekennzeichnet, dass** der Kondensator zwei vor der Benutzung, insbesondere während der Herstellung zugängliche Abschnitte (6) aufweist, die zur Aufladung des Kondensators dienen.

5. Kondom nach einem der Ansprüche 1 bis 4, **dadurch gekennzeichnet, dass** die leitfähigen Abschnitte (4) aus einer leitfähigen Schicht oder leitfähigen Folie, insbesondere aus Gold hergestellt, insbesondere auf die Schlauchhülle aufgedampft sind.

6. Verfahren zur Herstellung eines Kondoms gemäß einem der Ansprüche 1 bis 5, **dadurch gekennzeichnet, dass** die Schlauchhülle mit zwei leitfähigen Abschnitten (4) versehen wird, die den Kondensator bilden.

7. Verfahren nach Anspruch 6, **dadurch gekennzeichnet, dass** die leitfähigen Abschnitte (4) mittels Aufdampfen eines leitfähigen Stoffes, insbesondere eines Metalls, vorzugsweise Gold hergestellt werden.

8. Verfahren nach Anspruch 6 oder 7, **dadurch gekennzeichnet, dass** die Schlauchhülle an ihrer Innen- und/oder Außenseite mit Öffnungen (5) versehen wird.

9. Verfahren nach einem der Ansprüche 6 bis 8, **dadurch gekennzeichnet, dass** der Kondensator während der Herstellung aufgeladen wird.

10. Verfahren nach Anspruch 9, **dadurch gekennzeichnet, dass** Kondensator über die zugänglichen Abschnitte (6) aufgeladen wird.

## Claims

1. Condom consisting of a tubular sheath provided at one end with a tip (1) and at the opposite end with a bead (2) and made of a thin-walled, flexible insulating material (3), **characterized in that** the tubular sheath contains two conductive sections (4) which form a capacitor which is suitable for storing electric charge and for producing an electrical discharge during the use of the condom.

2. Condom according to Claim 1, **characterized in that** the tubular sheath is provided with openings (5), by way of which the capacitor can discharge during use.

3. Condom according to Claim 2, **characterized in that** the openings (5) are located on the inner side and/or on the outer side of the tubular sheath.

4. Condom according to one of Claims 1 to 3, **characterized in that** the capacitor has two sections (6) which are accessible prior to use, in particular during production, and serve for charging the capacitor.

5. Condom according to one of Claims 1 to 4, **characterized in that** the conductive sections (4) are produced from a conductive layer or a conductive foil, in particular from gold, and are applied to the tubular sheath by vapour deposition.

6. Method for producing a condom according to one of Claims 1 to 5, **characterized in that** the tubular sheath is provided with two conductive sections (4) which form the capacitor.

7. Method according to Claim 6, **characterized in that** the conductive sections (4) are produced by applying a conductive substance, in particular a metal, preferably gold, by vapour deposition.

8. Method according to Claim 6 or 7, **characterized in that** the tubular sheath is provided on its inner and/or outer side with openings (5).

9. Method according to one of Claims 6 to 8, **characterized in that** the capacitor is charged during production.

10. Method according to Claim 9, **characterized in that** the capacitor is charged via the accessible sections (6).

## Revendications

1. Préservatif constitué d'une enveloppe tubulaire munie à une extrémité d'une pointe (1) et à l'extrémité opposée d'un bourrelet (2), en un matériau isolant flexible à paroi mince (3), **caractérisé en ce que** l'enveloppe tubulaire contient deux sections conductrices (4) qui forment un condensateur, qui est approprié pour stocker une charge électrique et pour produire une décharge pendant l'utilisation du préservatif.

2. Préservatif selon la revendication 1, **caractérisé en ce que** l'enveloppe tubulaire est pourvue d'ouvertures (5), de sorte que le condensateur puisse se décharger pendant l'utilisation.

3. Préservatif selon la revendication 2, **caractérisé en ce que** les ouvertures (5) se situent au niveau du côté intérieur et/ou du côté extérieur de l'enveloppe tubulaire.

4. Préservatif selon l'une quelconque des revendications 1 à 3, **caractérisé en ce que** le condensateur présente deux sections (6) accessibles avant l'utilisation, notamment pendant la fabrication, qui servent à charger le condensateur.

5. Préservatif selon l'une quelconque des revendications 1 à 4, **caractérisé en ce que** les sections conductrices (4) sont fabriquées en une couche conductrice ou un film conducteur, notamment en or, notamment sont appliquées par vaporisation sur l'enveloppe tubulaire.

6. Procédé de fabrication d'un préservatif selon l'une quelconque des revendications 1 à 5, **caractérisé en ce que** l'enveloppe tubulaire est munie de deux sections conductrices (4) qui forment le condensateur.

7. Procédé selon la revendication 6, **caractérisé en ce que** les sections conductrices (4) sont fabriquées par vaporisation d'une substance conductrice, notamment d'un métal, de préférence de l'or.

8. Procédé selon la revendication 6 ou 7, **caractérisé en ce que** l'enveloppe tubulaire est munie d'ouvertures (5) sur son côté intérieur et/ou extérieur.

9. Procédé selon l'une quelconque des revendications 6 à 8, **caractérisé en ce que** le condensateur est chargé pendant la fabrication.

10. Procédé selon la revendication 9, **caractérisé en ce que** le condensateur est chargé par le biais des sections accessibles (6).
